Europäisches Patentamt

European Patent Office

Office européen des brevets

(19)

(11) Numéro de publication: **0 119 143**
**B1**

(12) **FASCICULE DE BREVET EUROPÉEN**

(45) Date de publication du fascicule du brevet:
23.11.88

(51) Int. Cl.⁴: **A 61 F 5/44**

(21) Numéro de dépôt: **84400512.4**

(22) Date de dépôt: **13.03.84**

(54) **Dispositif pour le recueil d'urines d'incontinents masculins.**

(30) Priorité: **14.03.83 FR 8304171**

(43) Date de publication de la demande:
**19.09.84 Bulletin 84/38**

(45) Mention de la délivrance du brevet:
**23.11.88 Bulletin 88/47**

(84) Etats contractants désignés:
**AT BE CH DE GB IT LI LU NL SE**

(56) Documents cité:
**EP-A-0 032 138**
**EP-A-0 094 614**
**WO-A-80/00535**
**FR-A-1 389 276**
**FR-A-2 396 541**
**GB-A-1 232 763**
**US-A-3 356 091**
**US-A-4 022 213**

(73) Titulaire: **LABORATOIRES BIOTROL S.A., 1, rue du Foin, F-75140 Paris Cedex 03 (FR)**

(72) Inventeur: **Demoulin, Bernard, Villa Tutina Vieille Route de Saint- Pee, F-64310 Saint- Pee S/ Nivelle (FR)**

(74) Mandataire: **Phélip, Bruno, c/o Cabinet Harlé & Phélip 21, rue de La Rochefoucauld, F-75009 Paris (FR)**

EP 0 119 143 B1

LIBER, STOCKHOLM 1988

## Description

La présente invention concerne un dispositif destiné à recueillir les urines chez les hommes, notamment chez les hommes souffrant d'incontinence, et plus particulièrement chez les incontinents urinaires masculins ambulatoires.

L'incontinence urinaire affecte une population non négligeable de patients. Elle peut être d'origine congénitale, mais aussi et surtout elle peut survenir à la suite d'un accident corporel ou d'une intervention chirurgicale ; en particulier les suites de traumatismes sévères de la colonne vertébrale et de la moelle épinière laissent apparaître fréquemment une inaptitude à l'émission volontaire des urines.

De plus, la situation et la relative immobilisation des individus devant rester alités ou de ceux qui sont grabataires implique qu'on recueille leurs urines artificiellement. De même, on peut rapprocher du traitement des incontinents urinaires le cas des personnes qui, bien que aptes à uriner normalement, se trouvent temporairement dans l'obligation de recourir à une collecte directe de leur urine dans un receptacle porté par elles.

On a déjà proposé un grand nombre de modalités pour la collecte des urines de patients de sexe masculin souffrant d'incontinence urinaire ou ne pouvant se lever. Ces modalités et les types de dispositifs correspondants peuvent se classer en trois catégories:

1. Dans le cas de patients atteints d'une incontinence partielle, ceux-ci utilisent seulement des couches absorbantes, telles que celles décrites par exemple dans le brevet FR-A-1 468 946.

Le maintien en place de ces couches est assuré par des culottes de divers types.

Mais ces couches, outre le fait qu'elles ne peuvent rendre service qu'à une infime minorité d'incontinents, présentent le défaut de provoquer rapidement des irritations dues au contact de l'épiderme avec les tissus imprégnés d'urine.

2. Dans les cas d'incontinence les plus sévères, c'est-à-dire quand les émissions d'urine peuvent être brutales et importantes, il est préconisé de collecter le liquide dans un sac vidangeable. Cela peut se faire au moyen d'etuis péniens, prolongés par un tube d'écoulement dont le but est de conduire l'urine jusqu'à un sac réservoir (voir en particulier les brevets FR-A-1 498 634, 1 508 356, 1 523 589, 1 591 685, 2 076 932, 2 083 122 et 2 364 645).

Ce type d'appareil est essentiellement constitué d'un condom en matière souple, notamment en latex, qui vient enserrer le pénis. La fixation d'un tel étui sur la verge est difficile, imparfaitement étanche, et peut provoquer des compressions excessives.

Dans certaines de leurs variantes, ces appareils impliquent même l'utilisation de produits adhésifs destinés au collage de l'étui sur la verge, et risquent de provoquer des irritations et des strictions de celle-ci.

3. Plus particulièrement pour des hommes actifs il est déjà apparu, dans le passé, préférable de réaliser un dispositif collecteur d'urine constitué d'un ensemble de plusieurs éléments dont la structure et l'agencement permettent une plus grande autonomie et un meilleur confort. Ce type d'appareillage se distingue des précédents en ce qu'il comprend, de manière générale, une partie fixée au corps, qui fait office de réceptacle et assure le joint avec la verge, et à laquelle est reliée une poche de recueil, généralement vidangeable.

Ainsi, on trouve décrit dans le brevet FR-A-1 219 897 un tel système comprenant une poche de recueil renfermant un matériau spongieux destiné à pomper les urines.

Par ailleurs, dans le brevet FR-A-2 242 964, on décrit un système simple rentrant également dans cette catégorie et comportant un diaphragme plastique destiné à enserrer la verge de façon étroite et une couche de matière collante pour maintenir la poche de recueil par adhérence sur la face extérieure du système.

On connaît également un dispositif pour le recueil des urines d'incontinents masculins, mis sur le marché sous la dénomination commerciale "Disposan", par la Société AB Medettprodukter, et qui peut être décrit comme comportant une ceinture avec lanières un premier anneau rigide, un second anneau semi-rigide, un manchon tronconique en latex et une poche de recueil vidangeable pour l'urine émise.

Le montage d'un tel dispositif implique que l'utilisateur doive retrousser l'extrémité libre de la poche sur le second anneau selon une configuration inverse de celle décrite dans le brevet FR-A-1 365 704, et ensuite emmancher le tout en force sur le rebord libre de la gorge radiale du premier anneau, tandis que le manchon élastique tronconique a au préalable été inséré dans celui-ci et fixé sur son rebord côté pubis.

La demande de brevet européen EP-A-0 032 138 décrit un dispositif pour recueillir les urines d'incontinents urinaires masculins, comportant, outre une ceinture et une pièce de matière déformable avec perforation circulaire, un premier anneau en matière plastique rigide, dont la paroi côté pubis, est apte à être retenue par la pièce de matière déformable après engagement dans la perforation circulaire de celle-ci; un manchon tronconique en latex dont l'extrémité de plus grand diamètre est retroussée et plaquée élastiquement sur la paroi côté pubis du premier anneau; un second anneau, semi-rigide, s'encliquetant sur le premier anneau; et une poche de recueil. Ce dispositif présente l'inconvénient de rester d'un montage assez compliqué en outre, le fait de prévoir des éléments rigides ou semi-rigides en rend le port gênant.

On connaît également, par le brevet des Etats-Unis d'Amerique US-A-3 356 091, un vêtement pour incontinents de la vessie. Un long tube en matière plastique souple, qui se termine dans un réservoir solidaire de la jambe du malade, est maintenu sur un slip par une gaine tissée qui est fixée sur ledit slip. Cet ensemble est rendu étanche par un joint en caoutchouc. Un tel dispositif n'est pas pratique; par ailleurs, étant réalisé sans la partie de recueil des urines amovible, il n est pas d'un entretien aisé.

La demande internationale WO-A-80/00535 décrit un slip pour incontinents masculins, comportant un orifice pour le passage de la verge, une poche de recueil en forme d'entonnoir fixé autour de l'orifice du slip par une pièce avant plate moulée d'une seule pièce avec ladite poche, et un manchon de guidage de la verge à la sortie du slip, se trouvant donc dans ladite poche de recueil. Une première couture réunit la pièce avant, à sa périphérie avec le slip, et une seconde couture, effectuée autour de l'ouverture du slip, réunit celui-ci, la poche et le manchon. On constate que ce slip n'est pas non plus d'un emploi et d'un entreiten aisés car la partie de rucueil n'est ras non plus amovible.

Le problème qui est à la base de la présente invention est la réalisation d'un dispositif efficace et aisé à utiliser pour le recueil de l'urine chez des sujets masculins dans des conditions aussi bonnes voire meilleures, que celles que permettent d'atteindre les dispositifs précités.

A cet effet, selon l'invention, on propose un dispositif pour le recueil d'urines chez l'homme, composé d'un slip à ouverture frontale sur lequel s'adapte une poche de recueil. La poche et le slip, auquel la poche peut être fixée, comportent autour de leurs orifices respectifs des éléments de fixation de type pour l'un à boucles et pour l'autre à crochets multiples et souples, de telle sorte que les deux parties soient rendues temporairement solidaires pour l'usage avec leurs deux orifices en coïncidence. L'originalité résulte de la présence d'un manchon associé de façon simple, par une collerette, au slip à ouverture frontale et/ou à la poche de recueil adaptable sur ce dernier.

Un tel manchon, notamment tronconique, de par sa nature et sa forme permet l'utilisation de l'invention par tous les patients incontinent urinaire, quelle que soit la forme et les dimensions de leur verge, y compris ceux qui ont une verge rétractée (pénis involué) et qui ne disposaient pas jusqu'alors d'un matériel adapté à leur morphologie.

La présente invention a donc pour objet un dispositif pour le recueil d'urines chez l'homme, composé d'un slip comportant, sur sa face avant, un orifice permettant le passage de la verge, d'une poche de recueil munie d'un orifice, et d'un manchon en matériau souple et/ou élastomère destiné à guider la verge, ce manchon étant pourvu à l'une de ses bases d'une collerette de fixation fixée à l'un desdits orifices, de façon à rendre le manchon coaxial à cet orifice, caractérisé par le fait que l'orifice de la poche est situé sur une face frontale de ladite poche et permet le passage de la verge, que ledit slip et ladite poche comportent, autour de leurs orifices respectifs, des éléments de fixation de type, pour l'un, à boucles, et, pour l'autre à crochets multiples et souples, de telle sorte que le slip et la poche soient rendues temporairement solidaires avec leurs deux orifices en coïncidence, que ledit manchon est de forme générale cylindrique ou tronconique, et que sa collerette est soit fixée autour dè l'orifice ménagé dans le slip sur le côté du slip se trouvant au contact de l'utilisateur ou entre le slip et l'élément de fixation qu'il porte, soit fixée sur la poche de recueil autour de l'orifice porté par celle-ci, sur le côté interne de la face frontale de la poche comportant l'orifice ou entre le côté externe et l'élément de fixation qui y est apposé.

Conformément à une caractéristique du dispositif selon la présente invention, l'élément de fixation de type à boucles est remplacé par un élément en tissu ou non-tissé, procurant avec l'élément de fixation de type à crochets une résistance d'accrochage du même ordre de grandeur que l'élément à boucles.

Par ailleurs, l'élément de fixation que comporte le dispositif selon l'invention a des dimensions d'ouverture supérieures à celles de l'orifice ménagé dans le tissu du slip.

De plus, on préfère que l élément de fixation ne soit pas solidaire de la poche de recueil sur toute sa surface.

La poche de recueil peut être vidangeable et comporter une valve anti-reflux.

La présente invention a également pour objet un slip comportant sur sa face avant un orifice permettant le passage de la verge, qui est caractérisé par le fait qu'autour de cet orifice, est fixé un élément de fixation de type à boucles ou à crochets multiples et souples de dimensions d'ouverture supérieures à celles dudit orifice, ainsi qu'un manchon souple par une collerette, selon ce qui a été indiqué ci-dessus.

En variante, le slip peut être un sous-vêtement plus enveloppant, par exemple du type caleçon à jambes courtes ou longues. Pour simplifier, on désignera uniquement par le terme de "slip" ce sous-vêtement dans le présent texte, étant entendu que cela désigne cependant toutes les variantes possibles d'un tel article.

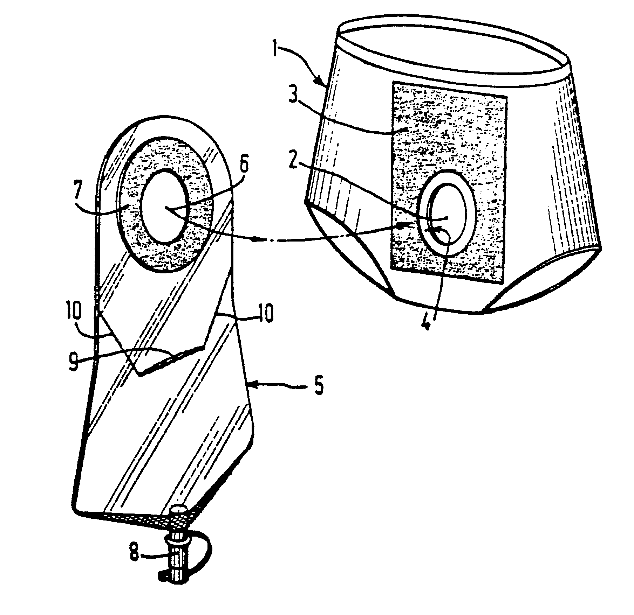

L'appareillage selon l'invention comporte d'autres caractéristiques et avantages, qui apparaîtront au cours de la description qui en est faite ci-après, à titre d'exemples non limitatifs en référence à la figure de la planche de dessin annexée, qui illustre, en perspective schématique et séparément, d'une part un slip et d'autre part une poche de recueil conformes à l'invention.

Le slip 1 peut être réalisé en une matière textile quelconque, faite de fibres synthétiques et/ou naturelles, ou même en non-tissé.

L'orifice 2 situé sur la face avant du slip peut être ménagé à la fabrication de celui-ci ou obtenu par découpe sur le slip fini ou en cours de confection. En pratique, il convient que cet orifice ait, sans que cela soit limitatif, un diamètre d'environ 30 mm.

Egalement sur la face avant du slip et à l'extérieur de celle-ci est apposé - par couture, collage, ou tout autre mode de fixation approprié - un élément de fixation 3 du type à boucles multiples et souples, notamment du type connu sous la dénomination commerciale Velcro et plus particulièrement du type Velcro dit Astrakan, composé d'une plaque ayant une forme rectangulaire, circulaire ou autre et comportant une découpe traversante ou orifice 4 ayant un diamètre sensiblement supérieur à celui de l'orifice du slip, avantageusement supérieur à 30 mm et de préférence d'environ 45 mm ou composé d'un ensemble de bandes disposées de façon à dégager un espace central correspondant à cet orifice ou dans lequel cet orifice peut s'inscrire.

A titre d'exemple, l'élément de fixation du type à boucles multiples et souples susdit peut être un rectangle d'environ 130 x 100 mm.

Il est clair que cet élément de fixation peut tout aussi bien être du type à crochets multiples et souples, auquel cas c'est alors l'élément apposé sur la poche de recueil qui est du type à boucles multiples et souples. Cette variante est cependant moins appréciée que la précédente, du fait qu'il existe alors un risque que lesdits crochets s'agrippent aussi dans d'autres vêtements tels qu'une chemise ou l'intérieur du pantalon de l'utilisateur.

En variante, dans un cas comme dans l'autre, il est également possible de substituer à l'élément de fixation du type à boucles multiples et souples un élément de fixation composé d'un tissu tissé ou non-tissé, par exemple en polyamide ou en une autre matière, pour autant qu'il procure, avec l'élément de fixation de type à crochets, une résistance d'accrochage du même ordre de grandeur que l'élément à boucles dont il constitue un équivalent technique.

Ce slip est destiné à être porté comme un slip classique par le patient, à qui il incombe simplement de faire sortir sa verge par ledit orifice, prévu à cet effet.

Le pénis du patient repose en partie sur le tissu du slip, ce qui lui assure un maximum de confort et empêche tout risque d'irritation.

La pièce rapportée 3 constituée par l'élément de fixation du type à boucles multiples et souples, outre sa fonction principale de fixation d'une poche de recueil munie elle-même d'un élément de fixation complémentaire, contribue également à conférer au slip une bonne tenue, lui permettant de résister aux sollicitations dues au poids de la poche remplie et préservant donc au patient son confort habituel.

Outre ce slip original, qui constitue lui-même un produit nouveau, le dispositif de recueil d'urines conforme à l'invention comprend une poche de recueil appropriée 5, à ouverture frontale 6, comportant sur la face extérieure munie de cette ouverture frontale, et solidaire de cette face extérieure, un élément de fixation 7 du type à crochets ou à boucles multiples et souples destiné à coopérer avec l'élément de fixation du type respectivement à boucles ou à crochets du slip susdit.

l'élément de fixation porte par la poche est complémentaire du premier, mais elle peut avoir une forme quelconque indépendante de celui-ci. De manière générale, la forme que peut présenter ce second élément de fixation peut être choisie à convenance parmi celles décrites plus haut pour le premier élément de fixation. En pratique, ce second élément est de préférence constitué par une couronne circulare, ayant avantageusement, mais non limitativement, des diamètres intérieur et extérieur d'environ 45 et 70 mm respectivement.

Le système selon l'invention procure une très grande facilité de mise en place et d'attache de la poche de recueil sur le slip porteur, même pour des personnes fébriles ou malhabiles.

La poche de recueil elle-même est de préférence en PVC; elle a avantageusement des dimensions de 250 mm environ pour la plus grande longueur et de 110 mm environ pour la plus grande largeur, et une capacité minimale d'environ 300 cm$^3$.

L'élément de fixation susdit étant convenablement fixé sur la poche autour de l'orifice frontal de celle-ci au moyen d'un adhésif approprié ou de tout autre moyen convenable. Dans une variante préférée, la couronne circulaire n'est plus fixée par toute sa surface à la poche, mais uniquement sur sa moitié intérieure, délimitée par un cercle concentrique à la perforation de diamètre intermédiaire entre les diamètres définissant la couronne.

Cette variante permet à la poche de bénéficier lors de son remplissage d'un degré de liberté supplémentaire dans la forme quelle peut prendre; les forces s'exerçant sur les liaisons couronne/poche et crochets/boucles ne sont plus appliquées perpendiculairement aux plans des liaisons mais forment avec lui un certain angle, ce qui fait que les liaisons sont soumises à des tensions plus faibles dans leur direction de moindre résistance. La résistance de l'acrochage réalisé en position d'utilisation entre les deux parties du système de fixation à boucles et crochets coopérants est de 1 à 2 daN pour une traction s'effectuant parallèlement entre les deux parties du système de fixation, alors que le poids maximum d'une poche pleine ayant les dimensions susdites est de 300 g environ.

D'autre part, l'utilisation d'une poche à ouverture frontale permet toute absence de qêne pour l'utilisateur, aussi bien lorsque la poche se gonfle au fur et à mesure de son remplissage que lors d'une érection dudit utilisateur.

Selon une variante préférée, la poche de recueil est du type vidangeable, c'est-à-dire une poche munie à son extrémité inférieure ou au voisinage de celle-ci d'un dispositif classique d'obturation et de vidange 8, soudé entre les deux films de la poche.

En variante, la poche de recueil peut également comporter - entre les deux films qui la composent et à un endroit tel que la verge du patient soit ainsi protégée sur toute sa longueur contre tout contact direct avec l'urine accumulée dans la poche - une valve antireflux 9, réalisée par tout moyen approprié, de préférence grâce à deux portions de films supplémentaires convenablement agencées et soudées l'une à l'autre selon des lignes 10 et entre les deux films constitutifs de la poche, par exemple comme décrit dans le document EP-A-0 032 138, auquel on peut utilement se référer. Le dispositif selon l'invention comporte un manchon (non représenté) en matériau souple et/ou élastomère, de forme générale cylindrique ou tronconique et comportant à sa base une collerette par exemple circulaire; ce manchon est fixé à demeure par sa collerette, par tout moyen approprié, autour de l'orifice ménagé dans le slip, soit sur le côté interne de la face avant du slip, soit entre le côté externe de la face avant du slip et l'élément de fixation qu'il porte, et s'étend coaxialement à l'orifice du slip vers l'extérieur de celui-ci. Avec le système de recueil d'urines ainsi équipé, l'utilisateur bénéficie d'un confort supplémentaire, pour autant que ce manchon souple et élastique, par exemple en latex ou en un autre matériau plastique adéquat, soit de dimensions appropriées pour épouser le contour de la verge de l'utilisateur sans créer de phénomène de strangulation et pour permettre à l'extrémité de la verge de déboucher hors de ce manchon.

Dans le cas où la collerette du manchon est fixée sur le côté du slip en contact avec la peau, il est avantageux de rec ouvrir cette collerette, sur sa partie restant visible après fixation, d'un matériau de revêtement plus approprié pour le contact avec le pubis du patient, en particulier d'un matériau non-tissé choisi parmi les non-tissés connus de l'homme du métier. L'apposition de ce matériau de recouvrement peut se faire par tout moyen approprié, notamment par collage. Ce matériau supplémentaire a pour fonction principale de protéger le patient contre les frottements du pubis sur le matériau de la collerette du manchon susdit.

En variante, le manchon susdit peut être dans la poche de recueil fixé autour de l'ouverture de celle-ci et concentriquement avec elle, soit sur le côte interne de la face de la poche comportant l'ouverture, soit entre le côté externe et l'élément de fixation qui y est apposé.

Dans tous les cas, les moyens de fixation du manchon sur la poche peuvent être le collage ou la soudure ou tout autre moyen approprié, en fonction de la nature des matériaux choisis.

**Revendications**

1. Dispositif pour le recueil d'urines chez l'homme, composé d'un slip comportant, sur sa face avant, un orifice permettant le passage de la verge, d'une poche de recueil munie d'un orifice, et d'un manchon en matériau souple et/ou élastomère destiné à guider la verge, ce manchon étant pourvu à l'une de ses bases d'une collerette de fixation fixée à l'un desdits orifices, de façon à rendre le manchon coaxial à cet orifice, caractérisé par le fait que l'orifice de la poche est situé sur une face frontale de ladite poche et permet le passage de la verge, que ledit slip et ladite poche comportent, autour de leurs orifices respectifs, des éléments de fixation de type, pour l'un à boucles, et, pour l'autre, à crochets multiples et souples, de telle sorte que le slip et la poche soient rendus temporairement solidaires avec leurs deux orifces en coincidence, que ledit manchon est de forme générale cylindrique ou tronconique et que sa collerette est soit fixée autour de l'orifice ménagé dans le slip sur le côté du slip se trouvant au contact de l'utilisateur ou entre le slip et l'élément de fixation qu'il porte, soit fixée sur la poche de recueil autour de l'orifice porté par celle-ci, sur le côté interne de la face frontale de la poche comportant l'orifice ou entre le côté externe et l'élément de fixation qui y est apposé.

2. Dispositif selon la revendication 1, caractérisé par le fait que l'élément de fixation de type à boucles est remplacé par un élément en tissu tissé ou non-tissé, procurant avec l'élément de fixation de type à crochets une résistance d'accrochage du même ordre de grandeur que l'élément à boucles.

3. Dispositif selon l'une de revendications 1 et 2, caractérisé par le fait que l'élément de fixation qu'il comporte à des dimensions d'ouverture supérieur à celles de l'orifice ménagé dans le tissu du slip.

4. Dispositif selon l'une de revendications 1 à 3, caractérisé par le fait que l'élément de fixation n'est pas solidaire de la poche de recueil sur toute sa surface.

5. Dispositif selon l'une des revendications 1 à 4, caractérisé par le fait que la poche de recueil est vidangeable et comporte une valve anti-reflux.

6. Slip comportant sur sa face avant un orifice permettant le passage de la verge, caractérisé par le fait qu'autour de cet orifice, est fixé un élément de fixation de type à boules ou à crochets multiples et souples de dimensions d'ouverture supérieures à celles dudit orifice, ainsi qu'un manchon souple par une collerette, selon la revendication 1.

## Patentansprüche

1. Vorrichtung zum Sammeln von Urinen bei Männern, bestehend aus einer Unterhose, die auf ihrem Vorderteil eine Öffnung aufweist, die die Durchführung des Penis erlaubt, aus einem mit einer Öffnung ausgestatteten Sammelbeutel und aus einer Hülle aus einem biegsamen und/oder elastomeren Material zur Führung des Penis, wobei die Hülle an einem ihrer Enden mit einem Befestigungsflansch versehen ist, der an einer der genannten Öffnungen derart befestigt ist, daß sich die Hülle koaxial zu dieser Öffnung befindet, dadurch gekennzeichnet, daß sich die Öffnung des Beutels auf einer Vorderseite des Beutels befindet und die Durchführung des Penis erlaubt, daß die Unterhose und der Beutel um ihre entsprechenden Öffnungen herum Befestigungselemente von der Art aufweisen, bei denen zum einen viele und biegsame Schlingen und zum anderen viele und biegsame Häkchen solcher Art vorhanden sind, daß die Unterhose und der Beutel zeitweise fest mit ihren beiden Öffnungen in Überdeckung zusammengehalten werden, daß die genannte Hülle im allgemeinen von zylindrischer oder konusstumpfförmiger Form ist und daß der Flansch der Hülle entweder um die in der Unterhose angebrachte Öffnung herum auf der Seite der Unterhose, die sich in Kontakt mit dem Benutzer befindet, oder zwischen der Unterhose und dem von ihr getragenen Befestigungselement befestigt ist oder daß der Flansch an dem Sammelbeutel um dessen Öffnung herum auf der Innenseite der die Öffnung aufweisenden Vorderwand des Beutels oder zwischen der Außenseite und dem Befestigungselement, das dort aufgedrückt ist, befestigt ist.

2. Vorrichtung nach Anspruch 1, dadurch gekennzeichnet, daß das Befestigungselement vom Schlingentyp durch ein gewebtes oder nichtgewebtes Stoffelement ersetzt ist, das mit dem Befestigungselement vom Häkchentyp einen Haftwiderstand der gleichen Größenordnung wie mit dem Schlingentypelement gewährleistet.

3. Vorrichtung nach einem der Ansprüche 1 und 2, dadurch gekennzeichnet, daß das Befestigungselement Öffnungsdimensionen aufweist, die größer sind als diejenigen der in dem Stoff der Unterhose angebrachten Öffnung.

4. Vorrichtung nach einem der Ansprüche 1 bis 3, dadurch gekennzeichnet, daß das Befestigungselement nicht auf seiner ganzen Fläche an dem Sammelbeutel befestigt ist.

5. Vorrichtung nach einem der Ansprüche 1 bis 4, dadurch gekennzeichnet, daß der Sammelbeutel entleerbar ist und ein Rückflußhinderungsventil aufweist.

6. Unterhose, die in ihrem Vorderteil eine Öffnung aufweist, die die Durchführung des Penis erlaubt, dadurch gekennzeichnet, daß um die Öffnung herum ein Befestigungselement des vielfachen und biegsamen Schlingentyps oder Häkchentyps mit je einer Durchlaßdimension, die größer ist als diejenige der genannten Öffnung, wie auch eine biegsame Hülle mit einem Flansch gemäß Anspruch 1 befestigt ist.

## Claims

1. Device for the collection of urines of males, consisting of briefs having, on their front face, an opening permitting the passage of the penis, a collecting bag equipped with an opening, and a muff of flexible and/or elastomeric material intended to guide the penis, this muff being provided at one of its bases with a fixing flange fixed to one of the said openings in such a way as to render the muff coaxial to this opening, characterized in that the opening of the bag is located on a front face of the said bag and permits the passage of the penis, in that the said briefs and the said bag comprise, around their respective openings, fixing elements of the type, on the one hand, with multiple and flexible loops and, on the other hand, with multiple and flexible hooks, so that the briefs and the bag are temporarily firmly attached with their two openings in coincidence, in that the said muff is of general cylindrical or truncated form, and in that its flange is either fixed around the opening made in the briefs on the side of the briefs in contact with the user or between the briefs and the fixing element which they bear, or is fixed on the collecting bag around the opening which the latter bears, on the inner side of the front face of the bag comprising the opening or between the outer side and the fixing element which is affixed thereto.

2. Device according to Claim 1, characterized in that the fixing element of the loop type is replaced by an element of woven or non-woven fabric, affording, with the fixing element of the hook type, a catching strength of the same order of magnitude as the loop element.

3. Device according to one of Claims 1 and 2, characterized in that the fixing element which it comprises has opening dimensions greater than those of the opening made in the fabric of the briefs.

4. Device according to one of Claims 1 to 3, characterized in that the fixing element is not firmly attached to the collecting bag over its complete surface.

5. Device according to one of Claims 1 to 4, characterized in that the collecting bag can be emptied and comprises an anti-reflux valve.

6. Briefs having, on their front face, an opening permitting the passage of the penis, characterized in that around this opening there is fixed a fixing element of the type with multiple and flexible loops or hooks of opening dimensions greater than those of the said opening, as well as a flexible muff by a flange, according to Claim 1.